(19) Europäisches Patentamt
European Patent Office
Office européen des brevets

(11) Veröffentlichungsnummer : **0 041 122**
**B1**

# (12) EUROPÄISCHE PATENTSCHRIFT

(45) Veröffentlichungstag der Patentschrift : **17.07.85**

(51) Int. Cl.[4] : **C 11 B 9/00**, A 23 L 1/226, C 07 D307/00

(21) Anmeldenummer : **81102983.4**

(22) Anmeldetag : **18.04.81**

## (54) Di- und Tetrahydrobenzofuranonderivate enthaltende Riechstoffkompositionen.

(30) Priorität : **03.05.80 DE 3017068**

(43) Veröffentlichungstag der Anmeldung : **09.12.81 Patentblatt 81/49**

(45) Bekanntmachung des Hinweises auf die Patenterteilung : **17.07.85 Patentblatt 85/29**

(84) *Benannte Vertragsstaaten :*
**CH DE FR GB LI NL SE**

(56) Entgegenhaltungen :
**US-A- 4 113 891**
**AGRICULTURAL AND BIOLOGICAL CHEMISTRY, Band 44, July 1980, Seiten 1535-1543 K. TAKAHASHI et al.: "A new keto-alcohol, (-) mintlactone, (+) isom ntlactone and minor components in peppermint oil"**
**AGRICULTURAL AND BIOLOGICAL CHEMISTRY, Band 37, Nr. 10, 1973, Seiten 2441,2442 I. SAKATA et al.: "Isolation and identification of 2,3-dimethyl 4 ydroxy 2 nonenoic acid lactone from Shubi"**
**JOURNAL OF THE AMERICAN CHEMICAL SOCIETY, Band 72, Januar 1950, Seiten 399-403 R.B. WOODWARD et al.: "The autoxidation of menthofuran"**
**S. Arctander, Perfume and Flower Materials of Natural Origin (1960) columns 513-516 and 659-660**
**H. Janistyn, Handbuch der Kosmetika und Riechstoffe, Band III (1950) Seiten 782 und 783**
**Die Akte enthält technische Angaben, die nach dem Eingang der Anmeldung eingereicht wurden und die nicht in dieser Patentschrift enthalten sind.**

(73) Patentinhaber : **HAARMANN & REIMER GMBH**
**Postfach 138**
**D-3450 Holzminden (DE)**

(72) Erfinder : **Köpsel, Manfred, Dr.**
**Schinkelstrasse 1**
**D-3450 Holzminden (DE)**
Erfinder : **Emberger, Roland, Dr.**
**Baerenfang 4**
**D-3450 Holzminden (DE)**

(74) Vertreter : **Mann, Volker, Dr. et al**
**c/o Bayer Aktiengesellschaft Zentralbereich Patente Marken und Lizenzen**
**D-5090 Leverkusen-Bayerwerk (DE)**

EP 0 041 122 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Di- und Tetrahydrobenzofuranonderivate enthaltende Riechstoffkompositionen sowie deren Verwendung.

Es wurde eine Riechstoffkomposition gefunden, die durch einen Gehalt von 1 bis 50 Gew.-% an Di- und Tetrahydrobenzofuranonderivaten der Formel

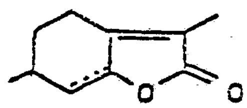

(I)

in der die gestrichelte Linie eine Einfach- oder Doppelbindung bedeutet, bezogen auf die gesamte Komposition, gekennzeichnet ist.

Die Verbindungen der allgemeinen Formel (I) 3,6-Dimethyl-5,6-dihydro-2(4H)-benzofuranon und 3,6-Dimethyl-5,6,7,7a-tetrahydro-2(4H)-benzofuranon sind literaturbekannt, ohne daß jedoch ihre Eignung als Riech- und Aromastoff erkannt worden ist. 3,6-Dimethyl-5,6-dihydro-2(4H)-benzofuranon, in der Literatur auch als Dehydromenthofurolacton bezeichnet, wurde durch Wasserabspaltung aus 7a-Hydroxi-3,6-dimethyl-4,5,6,7-tetrahydro-2-benzofuranon, dem Autoxidationsprodukt des Menthofurans hergestellt (J. Am. Chem. Soc. *72*, 399 (1950)). 3,6-Dimethyl-5,6,7,7a-tetrahydro-2(4H)-benzofuranon, in der Literatur auch Menthofurolacton genannt, wurde durch Hydrierung das Autoxidationsproduktes mit Natriumborhydrid hergestellt (Tetrahedron *23*, 2583 (1967)). Weiterhin wurden beide Verbindungen als Inhaltsstoffe des Pfefferminzöls nachgewiesen (Int. Congr. Essent. Oils, 6 th, *1974*, 142).

Die Herstellung der erfindungsgemäß zu verwendenden Verbindungen erfolgt nach folgendem Reaktionsschema :

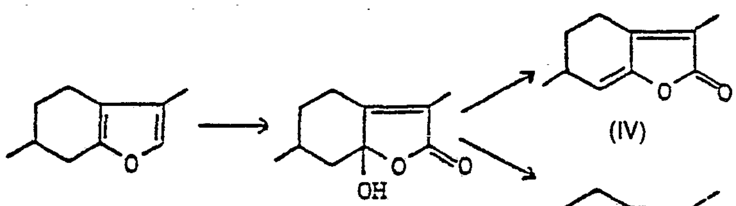

(II) (III) (IV) (V)

Menthofuran (II) wird in einer Photooxidationsreaktion mit Luftsauerstoff zu einem Hydroperoxid oxidiert, das in situ mit Natronlauge zu 7a-Hydroxi-3,6-dimethyl-4,5,6,7-terahydro-2-benzofuranon (III) zersetzt wird. Durch Wasserabspaltung z. B. mit Natriumhydrogensulfat (J. Am. Chem. Soc. *72*, 399 (1950)) wird aus dem Hydroximenthofurolacton (III) 3,6-Dimethyl-5,6-dihydro-2(H)-benzofuranon (IV) erhalten. Die Reduktion des Hydroximenthofurolactons (III) mit z. B. Natriumborhydrid (Tetrahedron *23*, 2583 (1967)) liefert 3,6-Dimethyl-5,6,7,7a-tetrahydro-2(4H)-benzofuranon (V).

Die erfindungsgemäß zu verwendenden Di- und Tetrahydrobenzofuranonderivate sind wertvolle Riechstoffe, die neben einer süß-lactonigen Note eine deutliche Cumarin- und Kokosnote aufweisen. Sie lassen sich sehr gut mit anderen Stoffen zu neuartigen Riechstoffkompositionen kombinieren, wobei sie in Mengen von 1 bis 50 Gewichtsprozent, bezogen auf die gesamte Komposition, eingesetzt werden können. Die neuen Kompositionen können zur Parfümierung von Kosmetika wie Cremes, Lotionen, Aerosolen, Toiletteseifen und technischen Produkten wie Wasch- und Reinigungsmitteln verwendet werden.

Weiterhin sind die erfindungsgemäß zu verwendenden Verbindungen wertvolle Aromastoffe, die sich aufgrund ihrer Cumarin-Note insbesondere zur Herstellung von Aromen mit Cumarin-, Waldmeister- und Karamelgeschmack eignen. Diese Eigenschaften sind deshalb besonders wichtig, weil die Verwendung von Cumarin selbst zur Aromatisierung von Lebensmitteln in vielen Ländern stark beschränkt ist und die erfindungsgemäß zu verwendenden Verbindungen dem derzeit gebräuchlichsten Cumarinersatzstoff Dihydrocumarin hoch überlegen sind.

Die unter Verwendung der Di- und Tetrahydrobenzofuranonderivate (III) und (IV) hergestellten Aromakompositionen können in dem gesamten Nahrungsmittelbereich, in dem Cumarin-/Waldmeister- und Karamelgeschmack erwünscht ist, eingesetzt werden. Insbesondere sind sie geeignet für Zuckersirup, Götterspeise, Erfrischungsgetränke, Speiseeis, Fondantmassen und ähnliches. Darüber hinaus können sie vorteilhaft als Cumarinersatz zur Tabakaromatisierung eingesetzt werden, da sie, genau wie Cumarin und cumarinhaltige Extrakte, z. B. Tonkabohnen- und Hirschzungenextrakt, dem Rauch nicht nur eine heu-waldmeisterartige Note verleihen, sondern ihn auch die unangenehme Schärfe nehmen und abrundend auf das Gesamtaroma wirken.

Die erfindungsgemäß zu verwendenden Verbindungen werden in Mengen von 0,001 bis 100 ppm, vorzugsweise 0,1 bis 10 ppm, bezogen auf das verzehrfertige Lebensmittel bzw. die fertige Tabakmischung verwendet.

Die nachfolgenden Beispiele sollen den Gegenstand der Erfindung näher erläutern, ohne ihn jedoch hierauf zu beschränken.

Beispiel 1

Eine Parfümkomposition mit Fougère-Note wird durch Mischen folgender Komponenten hergestellt :

200 Lavandin
100 Bergamotte Identoil
50 Linalylacetat
50 Citronenöl Identoil
100 Linalool
20 Geraniumöl Identoil
10 Rosmarinöl
10 Isoeugenol
50 Cedernholzöl
20 Patschuliöl
50 Sandel H + R
10 Eichenmoos absolue
5 Labdanum absolue
10 Zimtalkohol
10 Heliotropin
10 Vanillin
15 Moschus Keton
20 Moschus Ambrette
20 1,3,4,6,7,8-Hexahydro-4,6,6,7,8,8-hexamethyl-cyclopenta-γ-2-benzopyran
760 Gewichtsteile

Durch Zugabe von 50 Gewichtsteilen 3,6-Dimethyl-5,6-7-7a-tetahydro-2(4H)-benzofuranon wird die Komposition abgerundet und dadurch parfümistisch wertvoller und, bedingt durch den süßen Grundcharakter des Riechstoffes, gewinnt das Parfümöl an Charakter und Impact.

Beispiel 2

Durch Mischen der folgenden Komponenten wurde eine Grundmischung A mit Cumarin-Waldmeister-Geschmack hergestellt :

12 Aethylvanillin
60 Vanillin
60 Anisaldehyd
60 Methylcyclopentenolon
70 Heliotropin
738 Propylenglycol-1,2
1 000 Gewichtsteile

Mischung B wurde durch Zusatz von 190 Gewichtsteilen Dihydrocumarin unter gleichzeitiger Reduzierung des Propylenglycols auf 548 Gewichtsteile aus Mischung A erhalten.

Mischung C wurde durch Zusatz von 18 Gewichtsteilen 3,6-Dimethyl-5,6-dihydro-2(4H)-benzofuranon unter gleichzeitiger Reduzierung des Propylenglycols auf 720 Gewichtsteile aus Mischung A erhalten.

Mischung D wurde durch Zusatz von 18 Gewichtsteilen 3,6-Dimethyl-5,6,7,7a-tetrahydro-2(4H)-benzofuranon unter gleichzeitiger Reduzierung des Propylenglycols auf 720 Gewichtsteile aus Mischung A erhalten.

Die Mischungen A bis D wurden mit einer Dosierung von 20 ppm in einer 3,25 %igen Zuckerlösung in Wasser von einer Gruppe von Geschmacksprüfern verkostet. Die Beschreibung des Geschmackes der einzelnen Mischungen ergab :

A : leichter Cumarin-Waldmeister-Geschmack

B : Cumarin-Waldmeister-Geschmack

C : ausgeprägterer und typischerer Cumarin-Waldmeister-Geschmack als A und B, gleichzeitig weicher und voller.

D : ausgeprägterer und typischerer Cumarin-Waldmeister-Geschmack als A und B, gleichzeitig weicher und voller.

Beispiel 3

Mit den Mischungen A, C und D des Beispiels 2 wurde eine Feinschnittabakmischung mit einer Dosierung von 0,05 % aromatisiert. Beim sensorischen Test wurde bei der Verwendung der Mischungen C und D auf dem Tabak ein deutlich cumarinartiger Geruch festgestellt. Ebenso war im Rauch eine deutliche cumarinartige Geschmacksnote erkennbar. Gleichzeitig ist auch die abrundende und entschärfende Wirkung bei deisen Mischungen feststellbar.

Die Mischung A ergab eine wesentlich untypischere Cumarinnote und wirkte kaum abrundend und entschärfend.

Beispiel 4

Durch Mischen der folgenden Komponenten wurde eine Grundmischung E mit Karamelgeschmack hergestellt :

15 Diacetyl
15 Aethylvanillin
50 Heliotropin
80 Vanillin
80 Methylcyclopentenolon
760 Propylenglycol-1,2
1 000 Gewichtsteile

Mischung F wurde durch Zusatz von 2 Gewichtsteilen 3,6-Dimethyl-5,6-dihydro-2(4H)-benzofuranon unter gleichzeitiger Reduzierung des Propylenglycols auf 758 Gewichtsteile hergestellt.

Mischung G wurde durch Zusatz von 2 Gewichtsteilen 3,6-Dimethyl-5,6,7,7a-tetrahydro-2(4H)-benzofuranon unter gleichzeitiger Reduzierung des Propylenglycols auf 758 Gewichtsteile hergestellt.

Die Mischungen E bis G wurden mit einer Dosierung von 100 ppm in einer 3,25 %igen Zuckerlösung in Wasser von einer Gruppe von Geschmacksprüfern verkostet. Die Beschreibung des Geschmackes der einzelnen Mischungen ergab :

E : buttrig und leicht karamellig
F : typischer Karamelcharakter, wesentlich voller und kräftiger als E
G : typischer Karamelcharakter, wesentlich voller und kräftiger als E

**Patentansprüche**

1. Riechstoffkomposition, gekennzeichnet durch einen Gehalt von 1 bis 50 Gew.-% an Di- und Tetrahydrobenzofuranonderivaten der Formel

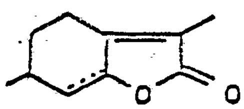

(I)

in der die gestrichelte Linie eine Einfach- oder Doppelbindung bedeutet, bezogen auf die gesamte Komposition.

2. Verwendung der Komposition nach Anspruch 1 als Riechstoff.

**Claims**

1. Perfume composition characterised in that it contains 1 to 50 % by weight of di- and tetrahydrobenzofuranone derivatives of the formula

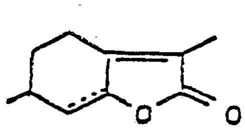

(I)

in which the broken line denotes a single or double bond, based on the total composition.

2. Use of the composition according to Claim 1 as a perfume.

**Revendications**

1. Composition de parfum, caractérisée par une teneur de 1 à 50 % en poids en dérivés de di- et tétrahydrobenzofurannone de formule

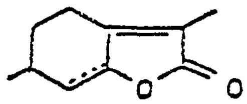

(I)

dans laquelle le segment en traits interrompus représente une liaison simple ou une liaison double, par rapport à la composition totale.

2. Utilisation de la composition suivant la revendication 1 comme parfum.